# EUROPEAN PATENT APPLICATION

(11) **EP 4 223 880 A1**
(43) Date of publication of application: **09.08.2023**
(21) Application number: 21875913.2
(22) Date of filing: 23.03.2021
(51) Int. Cl.: C12N 15/77, C12P 13/10

(54) **CORYNEBACTERIUM GLUTAMICUM VARIANT HAVING ENHANCED L-CITRULLINE PRODUCTION CAPACITY, AND METHOD FOR PRODUCING L-CITRULLINE USING SAME**

(30) Priority: 29.09.2020 KR 20200127021
(71) Applicant: Daesang Corporation, Seoul 02586 (KR)
(72) Inventor: LEE, Dong Seok, Yongin-si Gyeonggi-do 17063 (KR); CHOI, Min Jin, Daejeon 34116 (KR); KIM, Moon Jeong, Yeongcheon-si Gyeongsangbuk-do 38839 (KR); PARK, Seok Hyun, Namyangju-si Gyeonggi-do 12258 (KR); HAN, Jae Chun, Seoul 01913 (KR)
(74) Representative: Cabinet Nony
(86) International application number: PCT/KR2021/003602
(87) International publication number: WO 2022/071638

(57) **Abstract**

The present invention relates to a *Corynebacterium glutamicum* mutant strain having increased L-citrulline productivity and a method of producing L-citrulline using the same. The *Corynebacterium glutamicum* mutant strain is capable of producing L-citrulline in high yield and high concentration because the activity of the transport protein that is expressed by the NCgl2816 gene therein has been weakened or inactivated.

## Description

### Technical Field

The present invention relates to a *Corynebacterium glutamicum* mutant strain having increased L-citrulline productivity and a method of producing L-citrulline using the same.

### Background Art

L-citrulline is one of the non-essential amino acids, and it is known that citrulline has useful effects such as promoting ammonia metabolism, improving blood flow by vasodilation, lowering blood pressure, neurotransmission, enhancing immunity, and scavenging reactive oxygen species. Such citrulline is synthesized as an intermediate product during arginine biosynthesis. The biosynthesis of arginine in microorganisms proceeds from L-glutamate via eight enzymatic steps through two different pathways, a linear pathway and a cyclic pathway. In the linear pathway, L-arginine is synthesized from L-glutamate via N-acetylglutamate, N-acetylornithine, ornithine, citrulline, and argininosuccinate.

L-citrulline is generally produced by fermentation using microorganisms such as bacteria or yeast, and the production of L-citrulline may be performed using a naturally occurring wild-type strain or a mutant strain modified from the wild-type strain so as to have increased citrulline productivity. Recently, in order to improve the efficiency of production of L-citrulline, genetic recombination technology has been applied to microorganisms such as *Escherichia coli* and *Corynebacterium,* which have been widely used for the production of L-amino acids and other useful substances, and various recombinant strains or mutant strains having excellent L-citrulline productivity and methods of producing L-citrulline using the same have been developed. According to Korean Patent Nos. 10-1102263 and 10-1053429, it was confirmed that L-citrulline productivity is increased by enhancing or weakening the activities or expressions of proteins such as enzymes and transcription factors, which are involved in L-citrulline biosynthesis. Therefore, it may be expected that the production of L-citrulline may also be regulated by regulating the activities or expressions of various proteins involved in the L-citrulline biosynthesis pathway.

### [Prior Art Documents]

### [Patent Documents]

Korean Patent No. 10-1102263
Korean Patent No. 10-1053429

### DISCLOSURE

### Technical Problem

An object of the present invention is to provide a *Corynebacterium glutamicum* mutant strain having increased L-citrulline productivity.

Another object of the present invention is to provide a method of producing L-citrulline using the mutant strain.

### Technical Solution

The present inventors have conducted studies to develop a novel mutant strain having increased L-citrulline productivity using a *Corynebacterium glutamicum* strain, and as a result, have found that, when the NCg!2816 gene encoding a transporter or transport protein involved in the L-citrulline biosynthesis pathway is removed, L-citrulline production increases, thereby completing the present invention.

One aspect of the present invention provides a *Corynebacterium glutamicum* mutant strain in which the activity of a protein that is expressed by NCgl2816 gene has been weakened or inactivated and which has increased L-citrulline productivity.

As used herein, the term "NCgl2816 gene" refers to a gene encoding a putative integral membrane transport protein among various secondary transport proteins involved in active transport that overcome the concentration gradient between the outside and inside of the cell during substance movement through the cell membrane and selectively absorb or release nutrients.

According to one embodiment of the present invention, the NCgl2816 gene may be derived from a *Corynebacterium* sp. strain. Specifically, the *Corynebacterium* sp. strain may be *Corynebacterium glutamicum, Corynebacterium crudilactis, Corynebacterium deserti, Corynebacterium callunae, Corynebacterium suranareeae, Corynebacterium lubricantis, Corynebacterium doosanense, Corynebacterium efficiens, Corynebacterium uterequi, Corynebacterium stationis*, *Corynebacterium pacaense, Corynebacterium singulare, Corynebacterium humireducens, Corynebacterium marinum, Corynebacterium halotolerans, Corynebacterium spheniscorum, Corynebacterium freiburgense, Corynebacterium striatum, Corynebacterium canis, Corynebacterium ammoniagenes, Corynebacterium renale, Corynebacterium pollutisoli*, *Corynebacterium imitans, Corynebacterium caspium, Corynebacterium testudinoris, Corynebacaterium pseudopelargi* or *Corynebacterium flavescens,* without being limited thereto.

According to one embodiment of the present invention, the NCgl2816 gene may consist of the nucleotide sequence of SEQ ID NO: 1. According to another embodiment of the present invention, the NCgl2816 gene may encode the amino acid sequence of SEQ ID NO: 2.

According to one embodiment of the present invention, the protein expressed by the NCgl2816 gene is a transport protein involved in the L-citrulline biosynthesis pathway, and the effect of this transport protein on the production of L-citrulline has not been known or studied. However, the present inventors have found that, when the NCg!2816 gene encoding the transporter protein is deleted, the membrane stability may be improved, the release of L-citrulline may increase, the release of the intermediate 6-acetylornithine that is additionally detected in the overproduction of L-citrulline may be inhibited, and thus L-citrulline may be produced in high yield and high concentration.

As used in the present invention, "weakened activity" means that the expression level of a gene of interest has decreased compared to the original expression level. The term "weakened activity" also includes: a case in which the activity of the protein itself has decreased compared to the activity of the protein in the parent microorganism due to substitutions, insertions, deletions or combinations thereof, of one or more nucleotides in the nucleotide sequence encoding the gene; a case in which the overall activity of the enzyme in the cell is lower than that in the native strain, the wild-type strain, or the strain before modification, due to decreased expression or translation of the gene encoding the enzyme; and a combination thereof.

As used in the present invention, "inactivated" means that a gene encoding a protein such as an enzyme, a transcription factor or a transport protein is not expressed at all compared to that in the native strain, the wild-type strain, or the strain before modification, or that the gene has no activity even though it is expressed.

According to one embodiment of the present invention, a *Corynebacterium glutamicum* mutant strain having increased L-citrulline productivity compared to a wild-type *Corynebacterium glutamicum* strain or a *Corynebacterium glutamicum* mutant strain previously developed to overproduce citrulline has been obtained by deleting the NCgl2816 gene from the *Corynebacterium glutamicum* strain.

As used herein, the term "increased productivity" means that L-citrulline productivity has increased compared to that in the parent strain. As used herein, the term "parent strain" refers to a wild-type strain or mutant strain to be mutated, and includes a strain that is to be mutated directly or to be transformed with a recombinant vector or the like. In the present invention, the parent strain may be a wild-type *Corynebacterium glutamicum* strain or a strain mutated from the wild-type strain. For example, the parent strain may be a *Corynebacterium glutamicum* mutant strain which has enhanced activities of ornithine carbamoyltransferase and carbamoyl phosphate synthase to overproduce citrulline (see Korean Patent Application No. 10-2019-0151321).

According to one embodiment of the present invention, the *Corynebacterium glutamicum* mutant strain having increased L-citrulline productivity exhibits increased L-citrulline productivity compared to the parent strain. In particular, the amount of L-citrulline produced by the *Corynebacterium glutamicum* mutant strain may be at least 0.5%, specifically 0.5% to 5% higher than that produced by the parent strain, while the amount of 6-acetylornithine produced as a by-product may be at least 20%, specifically 20 to 50% lower than that produced by the parent strain.

The *Corynebacterium glutamicum* mutant strain according to one embodiment of the present invention may be obtained through a recombinant vector for deletion of the NCgl2816 gene in the parent strain.

As used herein, the term "vector" refers to any vehicle for the cloning of and/or transfer of a nucleic acid into a host cell. The vector may be a replicon to which another DNA fragment may be attached so as to bring about the replication of the attached fragment. The term "replicon" refers to any genetic element (e.g., plasmid, phage, cosmid, chromosome, virus) that functions as an autonomous unit of DNA replication *in vivo,* i.e., capable of replication under its own control. In the present invention, the vector is not particularly limited as long as it may replicate in a host, and any vector known in the art may be used. The vector used for the construction of the recombinant vector may be a plasmid, cosmid, virus or bacteriophage in a native state or a recombinant state. Example of a phage vector or cosmid vector that may be used include, but are not limited to, pWE15, M13, λEMBL3, λEMBL4, λFIXII, λDASHII, λZAPII, λgt10, λgt11, Charon4A, and Charon21A, and examples of a plasmid vector that may be used include, but are not limited to, a pDZ vector, and pBR-based, pUC-based, pBluescriptll-based, pGEM-based, pTZ-based, pCL-based and pET-based vectors.

As used herein, the term "transformation" means introducing a gene into a host cell so that the gene may be expressed in the host cell. The transformed gene may include any gene, regardless of whether the gene is inserted into the chromosome of the host cell or located outside of the chromosome, as long as the gene may be expressed in the host cell.

According to one embodiment of the present invention, the transformation method includes any method of introducing a nucleic acid into a cell, and may be performed using a suitable standard technique known in the art, selected depending on the host cell. Examples of the transformation method include, but are not limited to, electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method.

According to one embodiment of the present invention, as the transformation method, an electroporation method (van der Rest et al., Appl. Microbiol. Biotechnol., 52, 541-545, 1999) may be used.

The host cell includes a cell transfected, transformed, or infected with the recombinant vector or polynucleotide of the present invention *in vivo* or *in vitro.* The host cell including the recombinant vector of the present invention is a recombinant host cell, a recombinant cell, or a recombinant microorganism.

In addition, the recombinant vector according to the present invention may contain a selection marker. The selection marker is used to select transformants (host cells) transformed with the vector. Since only cells expressing the selection marker can survive in a medium treated with the selection marker, it is possible to select the transformed cells. Representative examples of the selection marker include, but are not limited to, kanamycin, streptomycin, chloramphenicol, and the like.

Genes inserted into the recombinant vector for transformation according to the present invention may be integrated into a host cell such as a microorganism, for example, a *Corynebacterium* sp. strain or *E*. *coli,* by homologous recombination crossover.

Another aspect of the present invention provides a method for producing L-citrulline comprising steps of: a) culturing the *Corynebacterium glutamicum* mutant strain in a medium; and b) recovering L-citrulline from the cultured mutant strain or the medium in which the mutant strain has been cultured.

The culturing may be performed using a suitable medium and culture conditions known in the art, and any person skilled in the art may easily adjust and use the medium and the culture conditions. Specifically, the medium may be a liquid medium, but is not limited thereto. Examples of the culturing method include, but are not limited to, batch culture, continuous culture, fed-batch culture, or a combination thereof.

According to one embodiment of the present invention, the medium should meet the requirements of a specific strain in a proper manner, and may be appropriately modified by a person skilled in the art. For the culture medium for the *Corynebacterium* sp. strain, reference may be made to, but not limited to, a known document (Manual of Methods for General Bacteriology, American Society for Bacteriology, Washington D.C., USA, 1981).

According to one embodiment of the present invention, the medium may contain various carbon sources, nitrogen sources, and trace element components. Examples of carbon sources that may be used include: saccharides and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture, without being limited thereto. Examples of nitrogen sources that may be used include peptone, yeast extract, meat extract, malt extract, corn steep liquor, soybean meal, urea, or inorganic compounds such as ammonium sulfate, ammonium chloride, ammonium phosphate, ammonium carbonate, and ammonium nitrate. The nitrogen sources may also be used individually or as a mixture, without being limited thereto. Examples of phosphorus sources that may be used include, but are not limited to, potassium dihydrogen phosphate or dipotassium hydrogen phosphate or the corresponding sodium-containing salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be used in the culture medium. The medium or individual components may be added to the culture medium batchwise or in a continuous manner by a suitable method during culturing, without being limited thereto.

According to one embodiment of the present invention, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present invention, in the step of recovering L-citrulline from the cultured mutant strain or the medium in which the mutant strain has been cultured, the produced L-citrulline may be collected or recovered from the medium using a suitable method known in the art depending on the culture method. Examples of the method for recovering L-citrulline that may be used include, but are not limited to, centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobicity and size exclusion), and the like.

According to one embodiment of the present invention, the step of recovering L-citrulline may be performed by centrifuging the culture medium at a low speed to remove biomass and separating the obtained supernatant through ion-exchange chromatography.

According to one embodiment of the present invention, the step of recovering L-citrulline may include a process of purifying L-citrulline.

### Advantageous Effects

The *Corynebacterium glutamicum* mutant strain according to the present invention is capable of producing L-citrulline in high yield and high concentration because the activity of the transport protein expressed by the NCgl2816 gene therein has been weakened or inactivated.

### Mode for Invention

Hereinafter, the present invention will be described in more detail. However, these descriptions are provided for illustrative purposes only to aid in the understanding of the present invention, and the scope of the present invention is not limited by these illustrative descriptions.

### Example 1. Analysis of transcriptome-level changes in citrulline-producing strains

### 1-1. Cell collection

In order to analyze transcriptome-level changes in citrulline-producing strains, cell collection was performed at the time of change in the growth stage of each strain.

First, each of a *Corynebacterium glutamicum* mutant strain (see Korean Patent Application No. 10-2019-0151321, hereinafter referred to as "CT4"), which has enhanced activity of ornithine carbamoyltransferase and carbamoyl phosphate synthase to overproduce citrulline, and a wild-type *Corynebacterium glutamicum* strain (ATCC13032), was inoculated in a citrulline seed medium and cultured at 30°C for 10 hours, and then 250 mL of each culture was inoculated in a 5-L culture medium. When the glucose contained in the initial medium was completely exhausted, an additional medium was added. At 13, 28, 35, and 68 hours of culture after seeding, cells were collected and used for preparation of transcriptome analysis samples. The compositions of the media used in this experiment are shown in Table 1 below.

**[Table 1]**

| | Composition (/L) |
|---|---|
| Citrulline seed medium | 2.67% glucose, 3.14% YPA, 0.1% KH₂PO₄, 0.1% K₂HPO₄, 10 ppm MNSO₄, 10 ppm FeSO₄, 100 µg/L biotin, 200 µg/L thiamin-HCl |
| 5-L culture medium | 8% glucose, 0.08% MgSO₄, 16 ppm FeSO₄, 8 ppm MnSO₄, 1.6 ppm CuSO₄, 1.6 ppm ZnSO₄, 32 ppm CaCl₂, 160 ppb biotin, 8 ppm thiamine-HCl, 0.4% HVP, 0.08% NaCl, 0.22% KH₂PO₄, 2.4% (NH₄)₂SO₄ |
| Additional medium | 52% glucose, 0.1% MgSO₄, 120 ppm CaCl₂, 160 ppb biotin, 16 ppm thiamine-HCl, 0.08% NH₄H₂PO₄, 4.4% (NH₄)₂SO₄ |

### 1-2. Preparation of transcriptome analysis samples

After collecting cells in Example 1-1 above, the cultured cells were prepared at the same concentration, and RNAs were extracted therefrom using Rneasy Mini Kit (QIAGEN, Germany) according to the manufacturer's instructions. The extracted RNAs were stored in liquid nitrogen and subjected to whole genome transcriptome analysis by Macrogen Co., Ltd.

### 1-3. Selection of valid gene candidates

Based on the results of the whole genome transcriptome analysis of *Corynebacterium glutamicum* performed in Example 1-2 above, 40 candidate genes, which had an RPKM value of more than 1,000 and whose expression levels increased rapidly in the latter half of fermentation or were at least three times higher than those in the wild-type strain, were first selected from among permeases and transporters. For reference, it is known that the wild-type *Corynebacterium glutamicum* strain (ATCC13032) has little ability to produce citrulline.

*Corynebacterium glutamicum* strains deficient in each of the 40 genes were inoculated in flask titer medium (see Table 2 below) and cultured at 200 rpm at 30°C for 30 hours. After completion of culture, each culture was diluted 100-fold with distilled water and filtered through a 0.45-µm filter, and then the concentrations of L-citrulline and the by-product 6-acetylornithine in each strain culture were measured by using high-performance liquid chromatography (HPLC) equipped with a column (DionexlonPac^{™} CS12A) and an ultraviolet detector (195 mm). Five candidate strains with low concentrations of 6-acetylornithine were further selected from among the strains deficient in each of the 40 candidate genes. The concentrations of L-citrulline and 6-acetylornithine in the cultures of the wild-type strain and the five selected candidate strains are shown in Table 3 below.

**[Table 2]**

| | Composition (/L) |
|---|---|
| Flask titer medium | 105.3 g (95%) glucose, 1 g MgSO₄, 4 g YPA, 0.8 g KH₂PO₄, 1.2 g Na₂HPO₄, 30 g (NH₄)₂SO₄, 20 mg Fe_{S}O₄, 20 mg MnSO₄, 10 mg ZnSO₄, 100 mg arginine, 100 µg biotin, 200 µg thiamine |

**[Table 3]**

| Strain | L-citrulline (%) | 6-acetylornithine (%) |
|---|---|---|
| CT4 strain | 2.10 | 0.36 |
| NCgl2816-deficient strain | 2.12 | 0.10 |
| NCgl205-deficient strain | 1.98 | 0.32 |
| NCgl0397-deficient strain | 0.80 | 0.08 |
| NCgl0258-deficient strain | 2.11 | 0.36 |
| NCgl1095-deficient strain | 2.00 | 0.35 |

As a result, it was shown that, in the NCgl2816 gene-deficient strain, the production of L-citrulline was the highest and, and at the same time, the production of 6-acetylornithine was the lowest. Accordingly, the NCgl2816 gene was selected as the gene to be deleted for increasing L-citrulline production in the strain.

### Example 2. Construction of NCgl2816 gene-deleted mutant strain

### 2-1. Construction of vector

A wild-type *Corynebacterium glutamicum* strain (ATCC13032) was used to construct a *Corynebacterium glutamicum* mutant strain.

First, chromosomal DNA was extracted from the wild-type strain using the Wizard Genomic DNA Purification Kit (Promega, USA). Using the extracted DNA as a template, PCR was performed using each of a set of primers 1 and 2 and a set of primers 3 and 4. The obtained PCR products were amplified again by crossover PCR using a set of primers 1 and 4 and then inserted into Hindlll and Xbal restriction enzyme sites of the recombinant vector pK19mobSacB. The resulting vector was named vector pK19ms/△NCgl2816. For construction of the vector, the primers shown in Table 4 were used.

**[Table 4]**

| Primer | Sequence (5'→3') | SEQ ID NO. |
|---|---|---|
| Primer 1 | tgatttacgccaagctccttatccg | 3 |
| Primer 2 | ggaggggttttacttagattggtgacctcttctctgaaac | 4 |
| Primer 3 | gtttcagagaagaggtcaccaatctaagtaaaacccctcc | 5 |
| Primer 4 | ccggggatcctctagcgac | 6 |

Using the above-described primers, PCR was performed under the following conditions. PCR was performed using a thermocycler (TP600, TAKARA BIO Inc., Japan) in a reaction solution containing 100 µM of each deoxynucleotide triphosphate (dATP, dCTP, dGTP, dTTP), 1 pM oligonucleotide, 10 ng of the chromosomal DNA of *Corynebacterium glutamicum* ATCC13032 as a template, and 1 unit of PrimeSTAR Max DNA polymerase (Takara, Japan). PCR was performed for 25 to 30 cycles, each consisting of (i) denaturation at 94°C for 30 sec, (ii) annealing at 58°C for 30 sec, and (iii) extension at 72°C for 1 to 2 min (a polymerization time of 2 min per kb).

The gene fragment prepared as described above was cloned into a pK19mobSacB vector by self-assembly cloning. The vector was transformed into *E*. *coli* DH5a which was then plated on an LB-agar plate containing 50 µg/ml of kanamycin, and cultured at 37°C for 24 hours. The finally formed colony was isolated and whether the insert was exactly present in the vector was checked. Next, the vector was isolated and used for recombination of the *Corynebacterium glutamicum* strain.

As a process commonly performed in the above method, the corresponding genes were amplified by PCR from the genomic DNA of *Corynebacterium glutamicum* ATCC13032 and inserted into the pK19mobSacB vector by the self-assembled cloning method according to the strategy, and the resulting plasmid was selected in *E coli* DH5a. In this case, in order to insert the amplified gene into the pK19mobSacB vector, a DNA ligation kit (Takara, Japan) and restriction enzymes Hindlll and Xbal (NEB, England) were used according to the manufacturer's provided buffers and protocols.

### 2-2. Construction of mutant strain

A *Corynebacterium glutamicum* mutant strain was constructed by introducing the pK19ms/ΔNCgl2816 vector constructed in Example 2-1 above into the mutant strain CT4 as a parent strain.

Specifically, the parent strain was first cultured in 100 ml of RG medium (containing 10 g/L beef extract, 40 g/L BHI and 30 g/L sorbitol), and 2.5 g/L glycine, 400 mg/L isoniazid and 0.1 ml/L Tween80 were added to the same medium. Next, the seed culture was inoculated so that the OD₆₁₀ value reached 0.3, and then it was cultured at 180 rpm at 30°C for 3 to 5 hours so that the OD₆₁₀ value reached 1.6 to 1.8. The culture was kept on ice for 30 minutes and then centrifuged at 3500 rpm at 4°C for 15 minutes. Next, the supernatant was discarded, and the precipitated parent strain was washed 4 times with a 10% glycerol solution, and finally resuspended in 0.5 ml of a 10% glycerol solution. Electroporation was performed using a Bio-Rad electroporator. The competent cells prepared by the above method were placed in an electroporation cuvette (0.2 mm), and the pK19ms/ΔNCgl2816 vector was added thereto, followed by electroporation under the conditions of 2.5 kV, 200 Ω and 12.5 µF. Immediately after completion of the electroporation, 1 ml of a regeneration medium (containing 18.5 g/l brain heart infusion and 0.5 M sorbitol) was added to the cells which were then heat-treated at 46°C for 6 minutes. Next, the cells were cooled at room temperature, transferred into a 15-ml cap tube, incubated at 30°C for 2 hours, and plated on a solid medium (containing 40 g/L brain heart infusion, 30 g/L D-sorbitol, 10 g/L beef extract, and 20 g/L agar) containing 30 µg/ml kanamycine. The cells were cultured at 30°C for 2 days to obtain colonies. Among the colonies in which primary homologous recombination was induced, colonies in which amplification was confirmed by PCR using primers 1 and 4 of Table 4 above under the same conditions as in Example 2-1 above were selected as primary recombinant strains and cultured in 2YT liquid medium (containing 16 g/L tryptone, 10 g/L yeast extract and 5 g/L NaCl) for 12 hours. Next, the cultured colonies were plated on 2YT-10% sucrose solid medium (containing 16 g/L tryptone, 10 g/L yeast extract, 5 g/L NaCl and 100 g/L sucrose) to induce secondary homologous recombination, thus removing the antibiotic marker. The colonies cultured on the 2YT-10% sucrose solid medium were patched on 2YT-Km medium (containing 16 g/L tryptone, 10 g/L yeast extract, 5 g/L NaCl and 30 µg/ml kanamycine), and a strain that was not resistant to kanamycin and could grow in the 10% sucrose medium was finally selected. Whether the NCgl2816 gene was removed was finally checked by performing PCR using primers 1 and 4 shown in Table 4 above under the same conditions as in Example 2-1, and the NCgl2816 gene-deleted strain was named CT5.

### Example 3. Evaluation of L-citrulline productivity of NCgl2816 gene-deleted mutant strain

The L-citrulline productivities of the citrulline-overproducing *Corynebacterium glutamicum* mutant strain (CT4) used as the parent strain and the NCgl2816 gene-deleted strain (CT5) constructed in Example 2 were compared.

Each strain was inoculated into citrulline seed medium and cultured at 30°C for 10 hours. Then, 250 mL of each culture was inoculated into 5-L culture medium. When the glucose contained in the initial medium was completely exhausted, an additional medium was added. The compositions of the media used in this experiment are shown in Table 1 above. After completion of culture, each culture was diluted 100-fold with distilled water and filtered through a 0.45-µm filter, and then the concentrations of L-citrulline and the by-product 6-acetylornithine in each strain culture were measured using high-performance liquid chromatography (HPLC) equipped with a column (DionexlonPac^{™} CS12A) and an ultraviolet detector (195 mm). The results are shown in Table 5 below.

**[Table 5]**

| Strain | L-citrulline (%) | 6-acetylornithine (%) |
|---|---|---|
| CT4 | 8.56 | 1.6 |
| CT5 | 8.73 | 0.7 |

As shown in Table 5 above, it was confirmed that the L-citrulline productivity of the NCgl2816 gene-deleted *Corynebacterium glutamicum* mutant strain CT5 increased by about 2% compared to that of the previously developed mutant strain CT4, whereas the production of the by-product 6-acetylornithine decreased by about 44%. These results suggest that, when the activity of the protein that is expressed by the NCgl2816 gene is weakened or inactivated, the L-citrulline productivity of the strain can be increased and the production of by-products can also be decreased, and thus the strain is capable of producing L-citrulline with high purity.

So far, the present invention has been described with reference to the preferred embodiments. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

## Claims

1. A *Corynebacterium glutamicum* mutant strain in which an activity of a protein that is expressed by NCgl2816 gene has been weakened or inactivated and which has increased L-citrulline productivity.

2. The *Corynebacterium glutamicum* mutant strain according to claim 1, wherein the NCgl2816 gene consists of the nucleotide sequence of SEQ ID NO: 1.

3. The *Corynebacterium glutamicum* mutant strain according to claim 1, wherein all or a portion of the NCgl2816 gene has been inserted, substituted or deleted.

4. A method for producing L-citrulline comprising steps of:
a) culturing the *Corynebacterium glutamicum* mutant strain of claim 1 in a medium; and
b) recovering L-citrulline from the cultured mutant strain or the medium in which the mutant strain has been cultured.
